# EUROPEAN PATENT APPLICATION

(11) **EP 4 851 667 A1**
(43) Date of publication of application: **09.12.2026**
(21) Application number: 26183397.4
(22) Date of filing: 05.06.2026
(51) Int. Cl.: G01S 7/52, A61B 8/00, G01S 15/88, G01S 15/89

(54) **ULTRASONIC SIGNAL PROCESSING APPARATUS, ULTRASOUND DIAGNOSTIC APPARATUS, ULTRASONIC SIGNAL PROCESSING METHOD, AND PROGRAM**

(30) Priority: 06.06.2025 JP 2025094979; 19.05.2026 JP 2026084245
(71) Applicant: Canon Kabushiki Kaisha, Tokyo 146-8501 (JP)
(72) Inventor: IWASAKI, Ryosuke, Tokyo, 146-8501 (JP)
(74) Representative: Marks & Clerk LLP

(57) **Abstract**

An ultrasonic signal processing apparatus according to an embodiment includes an acquirer, a synthesizer, and processing circuitry. The acquirer acquires, by transmitting/receiving ultrasonic waves with different waveforms a plurality of times in a same direction, a plurality of ultrasonic signals corresponding to the plurality of transmissions/receptions, respectively. The synthesizer generates a synthesized signal by synthesizing the plurality of ultrasonic signals acquired by the acquirer using a coefficient that varies in a depth direction prior to beamforming. The processing circuitry performs the beamforming on the synthesized signal generated by the synthesizer.

## Description

### CROSS REFERENCE TO RELATED APPLICATIONS

This application is based upon and claims the benefit of priority from the prior Japanese Patent Applications No. 2025-94979, filed on June 6, 2025, and No. 2026-84245, filed on May 19, 2026.

### FIELD

The embodiments disclosed in the present specification and in the drawings relate to an ultrasonic signal processing apparatus, an ultrasound diagnostic apparatus, an ultrasonic signal processing method, and a program.

### BACKGROUND

Conventionally, in ultrasound diagnostic apparatus, harmonic imaging, which involves visualizing a harmonic signal at twice the frequency of a transmitted wave, has been performed to improve image quality of an ultrasound image. In harmonic imaging, a decrease in the signal-to-noise (S/N) ratio (in other words, sensitivity) in deep tissue attributable to attenuation of a transmitted wave may cause contrast to decrease to below a level of a fundamental wave image.

A conventional technique is known for improving the S/N ratio in deep tissue by blending data based on a fundamental wave signal to generate images. In the conventional technique, a beamformed first-rate signal and a beamformed second-rate signal are coherently added together. The addition generates a harmonic signal. The second-rate signal is a signal obtained from a transmitted wave of which a phase is inverted relative to the first-rate signal. In addition, in the conventional technique, a fundamental wave signal is generated using at least one of the beamformed first-rate signal and the beamformed second-rate signal. Then, the fundamental wave signal is blended into the harmonic signal.

However, the conventional technique required that the first-rate signal and the second-rate signal be separately subjected to beamforming in order to generate a harmonic signal. In other words, it was necessary to perform beamforming on two images' worth of image data in order to construct one image. Given that beamforming needs to be performed twice in order to generate a harmonic signal, the conventional technique often causes an increase in processing load or, more specifically, computational load, of beamforming, resulting in a decrease in image frame rate. Therefore, there is a need to reduce the processing load of beamforming while improving the frame rate.

### SUMMARY OF INVENTION

An ultrasonic signal processing apparatus, comprising:
an acquirer configured to acquire, by transmitting/receiving ultrasonic waves with different waveforms a plurality of times in a same direction, a plurality of ultrasonic signals corresponding to the plurality of transmissions/receptions, respectively;
a synthesizer configured to generate a synthesized signal by synthesizing the plurality of ultrasonic signals acquired by the acquirer using a coefficient that varies in a depth direction before beamforming; and
a processing circuitry configured to perform the beamforming on the synthesized signal generated by the synthesizer.

The processing circuitry may be further configured to generate an ultrasound image based on the synthesized signal subjected to the beamforming.

The acquirer may be further configured to transmit/receive, in the plurality of transmissions/receptions, ultrasonic waves modulated with respect to previously transmitted/received ultrasonic waves by transmitting/receiving ultrasonic waves of which phases have been changed with respect to the previously transmitted/received ultrasonic waves.

The acquirer may be further configured to transmit/receive, in the plurality of transmissions/receptions, ultrasonic waves modulated with respect to previously transmitted/received ultrasonic waves by transmitting/receiving ultrasonic waves of which amplitudes have been changed with respect to the previously transmitted/received ultrasonic waves.

The synthesizer may be further configured to synthesize the plurality of ultrasonic signals by adding the plurality of ultrasonic signals.

The synthesizer may be further configured to synthesize the plurality of ultrasonic signals by subtracting the plurality of ultrasonic signals.

The synthesizer may be further configured to synthesize the plurality of ultrasonic signals using the coefficient so that proportions of a harmonic component and a fundamental wave component included in the synthesized signal vary depending on depth.

The synthesizer may be further configured to synthesize the plurality of ultrasonic signals so that a proportion of the fundamental wave component becomes higher than a proportion of the harmonic component in the synthesized signal of a deep portion but the proportion of the harmonic component becomes higher than the proportion of the fundamental wave component in the synthesized signal of a shallow portion.

The synthesizer may be further configured to synthesize the plurality of ultrasonic signals so that an order of the harmonics becomes lower in the synthesized signal of a deep portion but the order of the harmonics becomes higher in the synthesized signal of a shallow portion.

The synthesizer may be further configured to synthesize the plurality of ultrasonic signals by further using a gain.

The synthesizer may be further configured to synthesize the plurality of ultrasonic signals by subjecting at least one ultrasonic signal among the plurality of ultrasonic signals to weighting using the coefficient.

The synthesizer may be further configured to synthesize the plurality of ultrasonic signals by applying a gain to at least one ultrasonic signal among the plurality of ultrasonic signals.

The synthesizer may be further configured to synthesize the plurality of ultrasonic signals by further using a phase rotation of the ultrasonic signal.

The synthesizer may be further configured to synthesize the plurality of ultrasonic signals using the coefficient and the phase rotation so that proportions of a second-order harmonic component and a third-order harmonic component included in the synthesized signal vary depending on depth.

The synthesizer may be further configured to synthesize the plurality of ultrasonic signals so that a proportion of the second-order harmonic component becomes higher than a proportion of the third-order harmonic component in the synthesized signal of a deep portion but the proportion of the third-order harmonic component becomes higher than the proportion of the second-order harmonic component in the synthesized signal of a shallow portion.

The processing circuitry may be further configured to acquire a signal strength of the ultrasonic signal before being synthesized by the synthesizer, and
the synthesizer is further configured to change the coefficient according to the signal strength acquired by the processing circuitry.

The synthesizer may be further configured to change the coefficient so that, when the signal strength is high, depth increases when the proportion of the fundamental wave component included in the synthesized signal becomes higher than the proportion of the harmonic component included in the synthesized signal, and when the signal strength is low, the depth decreases when the proportion of the fundamental wave component becomes higher than the proportion of the harmonic component.

An ultrasound diagnostic apparatus, comprising:
the ultrasonic signal processing apparatus according to claim 1; and
an ultrasound probe used in a plurality of transmissions/receptions of the ultrasonic waves.

An ultrasonic signal processing method, comprising:
acquiring, by transmitting/receiving ultrasonic waves with different waveforms a plurality of times in a same direction, a plurality of ultrasonic signals corresponding to the plurality of transmissions/receptions, respectively;
generating a synthesized signal by synthesizing the plurality of acquired ultrasonic signals using a coefficient that varies in a depth direction before beamforming; and
performing the beamforming on the generated synthesized signal.

A program for causing a computer to execute:
a procedure for acquiring, by transmitting/receiving ultrasonic waves with different waveforms a plurality of times in a same direction, a plurality of ultrasonic signals corresponding to the plurality of transmissions/receptions, respectively;
a procedure for generating a synthesized signal by synthesizing the plurality of acquired ultrasonic signals using a coefficient that varies in a depth direction before beamforming; and
a procedure for performing the beamforming on the generated synthesized signal.

### BRIEF DESCRIPTION OF THE DRAWINGS

FIG. 1A is a block diagram showing a configuration example of an ultrasonic signal processing apparatus according to a first embodiment;
FIG. 1B is a block diagram showing a configuration example of an ultrasound diagnostic apparatus according to the first embodiment;
FIG. 2 is a flow chart showing an operation example of the ultrasound diagnostic apparatus according to the first embodiment;
FIG. 3 is a graph showing coefficients in the operation example of the ultrasound diagnostic apparatus according to the first embodiment;
FIG. 4 is a diagram showing a generation step of a synthesized signal in the operation example of the ultrasound diagnostic apparatus according to the first embodiment;
FIG. 5 is a flow chart showing an operation of an ultrasound diagnostic apparatus according to a first comparative example;
FIG. 6A is a diagram showing an ultrasound image in the operation example of the ultrasound diagnostic apparatus according to the first embodiment;
FIG. 6B is an explanatory diagram for describing a frame rate in the operation example of the ultrasound diagnostic apparatus according to the first embodiment;
FIG. 7 is a block diagram showing a configuration of an ultrasound diagnostic apparatus according to a modification of the first embodiment;
FIG. 8 is a flow chart showing an operation of the ultrasound diagnostic apparatus according to the modification of the first embodiment;
FIG. 9 is a diagram showing a correction step of coefficients in an operation example of the ultrasound diagnostic apparatus according to the modification of the first embodiment;
FIG. 10 is a flow chart showing an operation example of an ultrasound diagnostic apparatus according to a second embodiment;
FIG. 11 is a diagram showing an ultrasound image in the operation example of the ultrasound diagnostic apparatus according to the second embodiment;
FIG. 12 is a flow chart showing an operation example of an ultrasound diagnostic apparatus according to a third embodiment;
FIG. 13 is a flow chart showing an operation example of an ultrasound diagnostic apparatus according to a second comparative example; and
FIG. 14 is a diagram showing an ultrasound image in the operation example of the ultrasound diagnostic apparatus according to the third embodiment.

### DETAILED DESCRIPTION

Hereinafter, embodiments of an ultrasound diagnostic apparatus will be described with reference to the drawings. In the following description, constituent elements with substantially a same function and a same configuration will be denoted by a same reference sign and overlapping descriptions will only be given when necessary.

### (First embodiment)

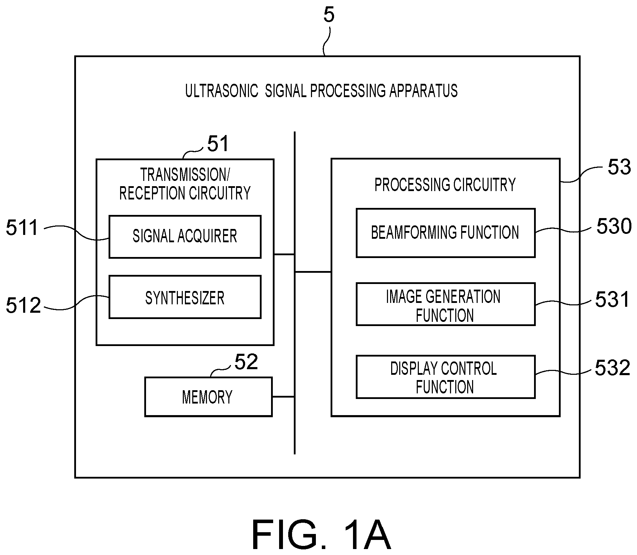

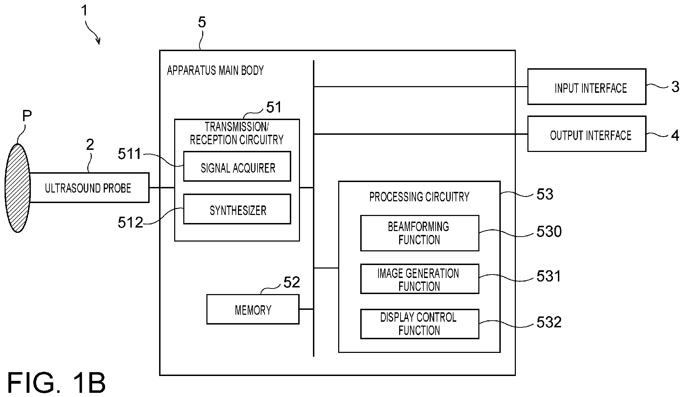

FIG. 1A is a block diagram showing a configuration example of an ultrasonic signal processing apparatus 5 according to a first embodiment. FIG. 1B is a block diagram showing an example of a configuration of an ultrasound diagnostic apparatus 1 according to the first embodiment. As shown in FIG. 1B, the ultrasonic signal processing apparatus 5 can be incorporated into the ultrasound diagnostic apparatus 1 in a mode of an apparatus main body 5 that constitutes the ultrasound diagnostic apparatus 1. Therefore, hereinafter, a configuration of the apparatus main body 5 will be described as a configuration of the ultrasonic signal processing apparatus 5 for convenience of description. However, specific modes of the ultrasonic signal processing apparatus 5 are not limited to the apparatus main body 5 shown in FIG. 1B.

As shown in FIG. 1B, the ultrasound diagnostic apparatus 1 according to the first embodiment includes an ultrasound probe 2, an input interface 3, an output interface 4, and the apparatus main body 5 that is an example of the ultrasonic signal processing apparatus 5. The ultrasound probe 2, the input interface 3, and the output interface 4 are communicably connected to the apparatus main body 5.

The ultrasound probe 2 is a device that transmits ultrasonic waves to a subject P and receives reflected ultrasonic waves (echoes) from the subject P in order to acquire an ultrasound image of the subject P.

The ultrasound probe 2 includes a plurality of transducers. The plurality of transducers generate ultrasonic waves based on a drive signal such as a drive voltage supplied from the apparatus main body 5. In addition, the ultrasound probe 2 receives reflected waves from a subject P and converts the reflected waves into an electric signal. In other words, the ultrasound probe 2 scans the subject P with ultrasonic waves and receives reflected waves from the subject P. The transducers are provided with electrodes for supplying the drive signal and for receiving the electric signal from the reflected waves. For example, the transducers may be constituted of lead zirconate titanate (PZT), polyvinylidene fluoride (PVDF), or the like. For example, an acoustic matching layer and an acoustic lens are arranged on a front surface of the transducers. For example, a backing material is arranged on a rear surface of the transducers. The acoustic matching layer is also referred to as a λ/4 layer and is a layer designed to efficiently transmit/receive ultrasonic waves by reducing an impedance difference between the transducer and biological tissue. The acoustic lens is a structure designed to minimize friction with biological surfaces during examination and to focus ultrasound beams to improve slice resolution. The backing material is a structure designed to absorb rearward ultrasonic waves and reduce a pulse width of forward ultrasonic waves. The ultrasound probe 2 is detachably connected to the apparatus main body 5.

When ultrasonic waves are transmitted from the ultrasound probe 2 to the subject P, the transmitted ultrasonic waves are reflected one after another at a discontinuity of acoustic impedance in a body tissue of the subject P and are received as a reflected wave signal by the plurality of the transducers included in the ultrasound probe 2. An amplitude of the received reflected wave signal depends on a difference in acoustic impedance at the discontinuity where the ultrasonic waves are reflected. The reflected wave signal when a transmitted ultrasound pulse is reflected by a surface such as a moving blood stream or a heart wall undergoes a frequency shift that depends on a velocity component relative to an ultrasound transmission direction of the mobile object due to a Doppler effect.

The ultrasound probe 2 is, for example, a 1D array probe that scans the subject P in two dimensions. The ultrasound probe 2 may also be a three-dimensional probe that scans the subject P in three dimensions or, in other words, a mechanical 4D probe or a 2D array probe.

The ultrasound probe 2 is, for example, a linear probe in which a biological contact surface on a probe head (not illustrated) that comes into contact with the subject P is formed flat along an alignment direction of the transducers. For example, the probe head includes the acoustic lens described above. However, the ultrasound probe 2 is not limited to a linear probe and may be, for example, a convex probe of which a biological contact surface is formed in a convex curved shape or a sector probe of which a biological contact surface is formed in a flat shape that is smaller than the linear probe.

The input interface 3 shown in FIG. 1B accepts various instructions and information input by an operator. Specifically, the input interface 3 converts an input operation accepted from the operator into an electric signal and outputs the electric signal to the apparatus main body 5. For example, the input interface 3 is realized by a trackball, a switch button, a mouse, a keyboard, a touch pad that allows an input operation to be performed by touching an operating surface, a touch screen that integrates a display screen and a touch pad, contactless input circuitry using an optical sensor, audio input circuitry, and the like. Note that the input interface 3 is not limited to those including physical operation components such as a mouse and a keyboard. For example, examples of the input interface 3 include electric signal processing circuitry that accepts an electric signal corresponding to an input operation from an external input device provided separately from the ultrasound diagnostic apparatus and outputs the electric signal to control circuitry.

The output interface 4 outputs various kinds of information. For example, the output interface 4 includes a display. The display converts information and image data sent from the apparatus main body 5 into electric signals for display and outputs the electric signals. The display is realized by a liquid crystal monitor, an organic light-emitting diode (OLED) monitor, a touch panel, and the like. The output interface 4 may include a speaker. The speaker outputs a predetermined sound such as a beep sound in order to notify a processing status of the apparatus main body 5 to the operator.

The apparatus main body 5 includes transmission/reception circuitry 51, a memory 52, and processing circuitry 53.

The transmission/reception circuitry 51 is circuitry that supplies drive signals to the ultrasound probe 2 under control of the processing circuitry 53. The transmission/reception circuitry 51 is also circuitry that generates reflected wave data by performing various kinds of processing on a reflected wave signal received by the ultrasound probe 2. For example, the transmission/reception circuitry 51 includes a processor. The transmission/reception circuitry 51 may include electric circuitry other than the processor.

The transmission/reception circuitry 51 includes a signal acquirer 511 and a synthesizer 512. The signal acquirer 511 is an example of the acquirer.

The signal acquirer 511 acquires, by transmitting/receiving ultrasonic waves with different waveforms a plurality of times in a same direction, a plurality of ultrasonic signals (in other words, received signals) corresponding to the plurality of transmissions/receptions, respectively.

In the plurality of transmissions/receptions, the signal acquirer 511 may transmit/receive ultrasonic waves modulated with respect to previously transmitted/received ultrasonic waves. More specifically, in the plurality of transmissions/receptions of ultrasonic waves, the signal acquirer 511 may transmit/receive ultrasonic waves of which phases have been changed (in other words, modulated) with respect to previously transmitted/received ultrasonic waves. For example, in the plurality of transmissions/receptions of ultrasonic waves, the signal acquirer 511 may transmit/receive ultrasonic waves of which phases have been changed by 180° (in other words, inverted) with respect to previously transmitted/received ultrasonic waves. Alternatively, in the plurality of transmissions/receptions of ultrasonic waves, the signal acquirer 511 may transmit/receive ultrasonic waves of which amplitudes have been changed with respect to previously transmitted/received ultrasonic waves.

For example, the signal acquirer 511 may acquire a first-rate received signal by a transmission/reception of a first-rate ultrasonic wave and, subsequently, acquire a second-rate received signal by a transmission/reception of a second-rate ultrasonic wave of which a phase or an amplitude has been changed with respect to the first-rate ultrasonic wave. A received signal is a channel signal representing a reflected wave per channel or per transducer.

The synthesizer 512 generates a synthesized signal by synthesizing a plurality of ultrasonic signals acquired by the signal acquirer 511 using a coefficient that varies in a depth direction before beamforming (in other words, in a stage of channel signals).

When transmitting/receiving an ultrasonic wave having been modulated with respect to a previously transmitted/received ultrasonic wave in the plurality of transmissions/receptions, the synthesizer 512 synthesizes a plurality of ultrasonic signals by adding the plurality of ultrasonic signals. The synthesizer 512 may synthesize a plurality of ultrasonic signals by subtracting the plurality of ultrasonic signals. When generating a synthesized signal by subtraction, modulation between previous and subsequent ultrasonic waves in the plurality of transmissions/receptions may be omitted.

The synthesizer 512 may synthesize a plurality of ultrasonic signals using a coefficient so that proportions of a harmonic component and a fundamental wave component included in the synthesized signal vary depending on depth.

Specifically, the synthesizer 512 may synthesize a plurality of ultrasonic signals so that a proportion of the fundamental wave component becomes higher than a proportion of the harmonic component in a synthesized signal of a deep portion. In addition, the synthesizer 512 may synthesize a plurality of ultrasonic signals so that a proportion of the harmonic component becomes higher than a proportion of the fundamental wave component in a synthesized signal of a shallow portion.

In addition, the synthesizer 512 may synthesize a plurality of ultrasonic signals so that an order of harmonics becomes lower in a synthesized signal of a deep portion but the order of harmonics becomes higher in a synthesized signal of a shallow portion.

In addition, the synthesizer 512 may synthesize a plurality of ultrasonic signals by subjecting at least one ultrasonic signal among the plurality of ultrasonic signals to weighting using a coefficient.

To further elaborate on the transmission/reception circuitry 51, for example, the transmission/reception circuitry 51 includes a pulse generator, a transmission delayer, and a pulser for supplying drive signals to the ultrasound probe 2. The pulse generator repeatedly generates rate pulses for forming transmission ultrasonic waves at a predetermined rate frequency. In addition, the transmission delayer focuses the ultrasonic waves generated by the ultrasound probe 2 into a beam and supplies a delay time required for each transducer to determine transmission directionality to each rate pulse generated by the pulse generator. The pulser applies a drive signal (drive pulse) to the ultrasound probe 2 at timings based on the rate pulses to which the delay times have been supplied. In other words, the transmission delayer arbitrarily adjusts a transmission direction of the ultrasonic waves transmitted from a transducer surface by varying the delay time supplied to each rate pulse. The transmission/reception circuitry 51 may transmit continuous-wave drive signals instead of pulsed drive signals. The pulse generator, the transmission delayer, and the pulser may constitute a part of the signal acquirer 511.

In addition, for example, the transmission/reception circuitry 51 includes a pre-amplifier, an analog/digital (A/D) converter, a reception delayer, and the synthesizer 512 in order to generate reflected wave data by performing various kinds of processing on a reflected wave signal received by the ultrasound probe 2. The pre-amplifier amplifies a reflected wave signal for each channel. The A/D converter subjects the amplified reflected wave signal to A/D conversion. The reception delayer supplies a delay time required to determine reception directionality. The pre-amplifier, the A/D converter, and the reception delayer may constitute a part of the signal acquirer 511. The configuration of the synthesizer 512 is as described earlier. Various modes can be selected as a mode of output signals from the transmission/reception circuitry 51 including signals containing phase information referred to as radio frequency (RF) signals and signals containing amplitude information after envelope detection processing.

In the example shown in FIG. 1B, the transmission/reception circuitry 51 is arranged in the apparatus main body 5. The transmission/reception circuitry 51 is not limited to being arranged in the apparatus main body 5 and at least a part of the transmission/reception circuitry 51 may be arranged in the ultrasound probe 2. In other words, at least parts of the signal acquirer 511 and the synthesizer 512 may be arranged in the ultrasound probe 2.

The memory 52 is a non-volatile storage device that stores various kinds of information, such as a hard disk drive (HDD), an optical disk, a solid-state drive (SSD), and an integrated circuit memory device. For example, the memory 52 stores a control program for controlling the ultrasound diagnostic apparatus 1 and various kinds of data used to execute the control program. In addition to an HDD, an SSD and the like, the memory 52 may also be a drive device that writes and reads various kinds of information to and from portable storage media such as a compact disc (CD), a digital versatile disc (DVD), and a flash memory or semiconductor memory devices such as a random access memory (RAM).

The processing circuitry 53 is circuitry that controls operations of the ultrasound diagnostic apparatus 1 as a whole in response to electric signals of input operations input from the input interface 3. For example, the processing circuitry 53 includes a beamforming function 530, an image generation function 531, and a display control function 532.

In this case, for example, each of the processing functions executed by the beamforming function 530, the image generation function 531, and the display control function 532 which are constituent elements of the processing circuitry 53 shown in FIG. 1B is recorded in the memory 52 in the form of a computer-executable program. The processing circuitry 53 is, for example, a processor. The processor that constitutes the processing circuitry 53 reads each program from the memory 52 and realizes a function corresponding to each read program by executing the program. In other words, the processing circuitry 53 in a state of having read each program is to include each function shown in the processing circuitry 53 in FIG. 1B.

While FIG. 1B shows a case where each of the processing functions of the beamforming function 530, the image generation function 531, and the display control function 532 is realized by the single processing circuitry 53, the embodiment is not limited thereto. For example, the processing circuitry 53 may be configured as a combination of a plurality of independent processors which realizes each processing function by having each processor execute each program. In addition, each processing function included in the processing circuitry 53 may be realized by being appropriately integrated to a single processing circuitry or distributed among a plurality of processing circuitries.

The beamforming function 530 may be included in the processor of the transmission/reception circuitry 51 instead of being included in the processing circuitry 53.

The beamforming function 530 performs beamforming on the synthesized signal generated by the synthesizer 512. The beamforming function 530 performs beamforming by, for example, supplying a delay time to the synthesized signal. The beamforming function 530 may perform phase modulation that involves modulating a phase of the synthesized signal. Performing phase modulation enables image blur to be suppressed and image quality to be enhanced.

The image generation function 531 generates an ultrasound image based on the synthesized signal subjected to beamforming by the beamforming function 530.

The image generation function 531 generates or, in other words, acquires the ultrasound image in response to scanning of the subject P using the ultrasound probe 2. For example, the image generation function 531 receives reflected wave data from the transmission/reception circuitry 51, performs envelope detection processing, logarithmic amplification processing, and the like, and generates data (B-mode data) where signal strength is expressed in terms of brightness. In addition, the image generation function 531 performs frequency analysis on velocity information from the reflected wave data received from the transmission/reception circuitry 51, extracts blood flow, tissue, and contrast agent echo components based on the Doppler effect, and generates data (Doppler data) representing extracted mobile object information such as velocity, dispersion, and power at multiple points. Furthermore, the image generation function 531 may be capable of processing both two-dimensional reflected wave data and three-dimensional reflected wave data. In other words, the image generation function 531 may generate two-dimensional B-mode data from two-dimensional reflected wave data and generate three-dimensional B-mode data from three-dimensional reflected wave data. In addition, the image generation function 531 may generate two-dimensional Doppler data from two-dimensional reflected wave data and three-dimensional Doppler data from three-dimensional reflected wave data.

In addition, the image generation function 531 generates a B-mode image representing an intensity of reflected waves as brightness from B-mode data. Furthermore, for example, the image generation function 531 generates a Doppler image that visualizes blood flow information from Doppler data. The Doppler image is velocity image data representing an average velocity of blood flow, dispersion image data representing a dispersion value of blood flow, power image data representing power of blood flow, or image data that combines these pieces of image data. In addition, as the Doppler image, the image generation function 531 generates a color Doppler image in which blood flow information such as the average velocity, the dispersion value, and the power of blood flow is displayed in color or generates a Doppler image in which one piece of blood flow information is displayed in grayscale. Furthermore, for example, the image generation function 531 can also generate an M-mode image from time-series data of B-mode data on one scan line. In addition, the image generation function 531 can also generate Doppler waveforms by plotting velocity information of blood flow and tissue over time from Doppler data.

The display control function 532 causes the display in the output interface 4 to display the ultrasound image generated by the image generation function 531.

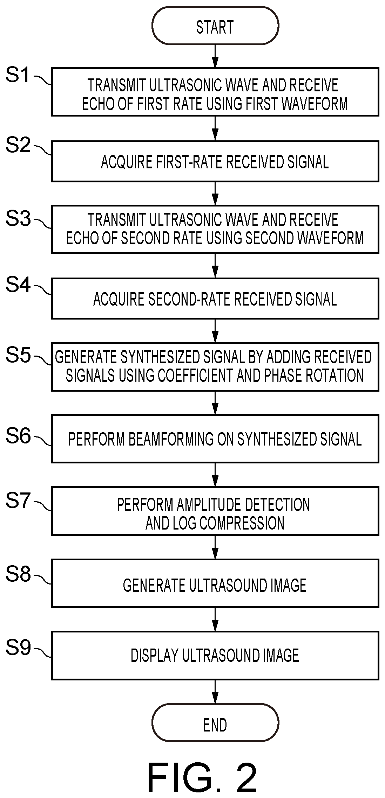

Next, an operation example of the ultrasound diagnostic apparatus 1 according to the first embodiment configured as described above will be described. FIG. 2 is a flow chart showing an operation example of the ultrasound diagnostic apparatus 1 according to the first embodiment.

First, as shown in FIG. 2, the signal acquirer 511 transmits an ultrasonic wave and receives an echo of a first rate using a first waveform via the ultrasound probe 2 (step S1).

After transmitting the ultrasonic wave and receiving the echo of the first rate, the signal acquirer 511 acquires a first-rate received signal (in other words, a channel signal) based on the received echo (step S2).

After acquiring the first-rate received signal, the signal acquirer 511 transmits an ultrasonic wave and receives an echo of a second rate using a second waveform via the ultrasound probe 2 (step S3).

After transmitting the ultrasonic wave and receiving the echo of the second rate, the signal acquirer 511 acquires a second-rate received signal based on the received echo (step S4).

After acquiring the second-rate received signal, the synthesizer 512 generates a synthesized signal by adding the first-rate received signal and the second-rate received signal using a coefficient that varies in the depth direction (step S5).

At this point, the synthesizer 512 adds the first-rate received signal and the second-rate received signal using the coefficient so that proportions of a harmonic component and a fundamental wave component included in the synthesized signal vary depending on depth.

Specifically, the synthesizer 512 adds the first-rate received signal and the second-rate received signal so that a proportion of the fundamental wave component becomes higher than a proportion of the harmonic component in a synthesized signal of a deep portion. In addition, the synthesizer 512 adds the first-rate received signal and the second-rate received signal so that the proportion of the harmonic component becomes higher than the proportion of the fundamental wave component in a synthesized signal of a shallow portion.

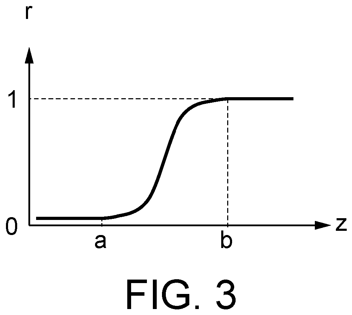

FIG. 3 is a graph showing a coefficient r in the operation example of the ultrasound diagnostic apparatus 1 according to the first embodiment. More specifically, the synthesizer 512 uses the coefficient r (in other words, a weight coefficient) shown in FIG. 3 to add (in other words, weighed addition) the first-rate received signal and the second-rate received signal. In the example shown in FIG. 3, the coefficient r has a value that varies in a depth direction z.

In the example shown in FIG. 3, the synthesizer 512 adds the first-rate received signal and the second-rate received signal according to the mathematical expression below. [Expression 1] $\begin{matrix}{s}_{sum} \left(z, θ, m\right) = {s}_{1} \left(z, θ, m\right) + \left(1-r\left(z\right)\right) ∗ {s}_{2} \left(z, θ, m\right) \\ r \left(z\right) = \left\{\begin{matrix}0 & \left(z<a\right) \\ 1 - \frac{1 + cos \left(\frac{z - a}{b - a}∗π\right)}{2} & \left(a\leq z\leq b\right) \\ 1 & \left(z>b\right)\end{matrix}\right.\end{matrix}$

In the mathematical expression presented above, reference sign sₛᵤₘ denotes an amplitude of the synthesized signal or, in other words, an amplitude of the ultrasonic signal after addition by the synthesizer 512 (the same shall apply hereinafter). Reference sign s₁ denotes an amplitude of the first-rate received signal or, in other words, an amplitude of the ultrasonic signal before addition by the synthesizer 512 that corresponds to a first transmission/reception (the same shall apply hereinafter). Reference sign s₂ denotes an amplitude of the second-rate received signal or, in other words, an amplitude of the ultrasonic signal before addition by the synthesizer 512 that corresponds to a second transmission/reception (the same shall apply hereinafter). Reference sign z denotes depth (the same shall apply hereinafter). Reference sign θ denotes a beam number of a beam obtained by a phased addition of received signals of a plurality of channels (the same shall apply hereinafter). Reference sign m denotes a channel number (the same shall apply hereinafter). Reference sign r denotes a coefficient (the same shall apply hereinafter). Reference signs a and b (where b > a) denote depths when a mode of transition of coefficients according to depth changes (the same shall apply hereinafter). In other words, a and b denote depths when an interval of the coefficient changes. The reference signs (z, θ, m) attached to an amplitude of a signal signify that the amplitude of the signal has a value corresponding to a combination of the depth z, the beam number θ, and the channel number m. The reference sign (z) attached to the coefficient signifies that the coefficient has a value corresponding to the depth z.

According to the above mathematical expression, the coefficient r is 0 in an interval where the depth is less than a. In this case, since sₛᵤₘ = s₁ + (1-0)s₂, the synthesized signal is a sum of the first-rate received signal and the second-rate received signal. Consequently, in shallow intervals, only the harmonic components (s₁ + s₂) are present.

In addition, according to the above mathematical expression, the coefficient r is 1-(1 + cos((z-a)/(b-a)*n))/2 in an interval where the depth is equal to or more than a and equal to or less than b. In other words, in an interval where the depth is equal to or more than a and equal to or less than b, the coefficient r takes values equal to or more than 0 and equal to or less than 1 and increases as the depth increases. In an interval where the depth is equal to or more than a and equal to or less than b, since sₛᵤₘ = s₁ + s₂ - rs₂, the interval contains a mixture of the harmonic components (s₁ + s₂) and the fundamental wave component (s₂) multiplied by an inverse gain (-r) or, in other words, -rs₂.

Furthermore, according to the above mathematical expression, the coefficient r is 1 in an interval where the depth exceeds b. In this case, since sₛᵤₘ = s₁ + (1-1)s₂, the synthesized signal is the first-rate received signal or, in other words, the fundamental wave signal. Consequently, in deep intervals, only the fundamental wave component is present.

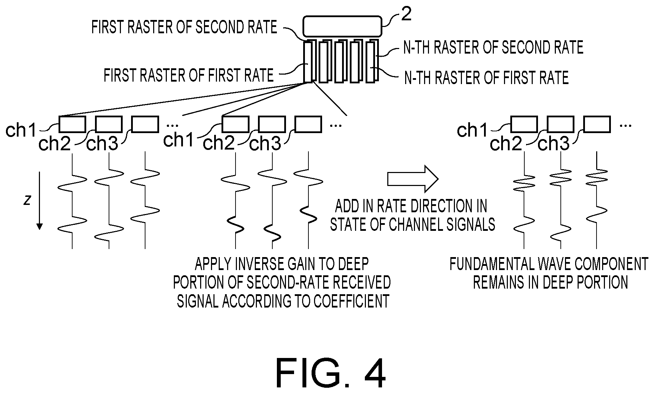

FIG. 4 is a diagram showing a generation step of a synthesized signal in the operation example of the ultrasound diagnostic apparatus 1 according to the first embodiment. The generation of a synthesized signal according to the above mathematical expression corresponds to the processing shown in FIG. 4. In the example shown in FIG. 4, for each channel (ch1, ch2, ch3, ...), the synthesizer 512 multiplies a deep portion by an inverse gain according to the coefficient with respect to the second-rate received signal. After multiplying the inverse gain, for each channel, the synthesizer 512 adds up the first-rate received signal itself and the second-rate signal of which the deep portion has been multiplied by the inverse gain in a state of a channel signal. In other words, the synthesizer 512 adds up the first-rate received signal itself and the second-rate received signal of which the deep portion has been multiplied by the inverse gain in a rate direction. The addition generates a synthesized signal in which the fundamental wave component remains in a deep portion.

After the synthesized signal is generated, as shown in FIG. 2, the beamforming function 530 performs beamforming on the synthesized signal (step S6).

After beamforming is performed, the processing circuitry 53 performs amplitude detection and log compression with respect to the beamformed synthesized signal (step S7).

After amplitude detection and log compression are performed, the image generation function 531 generates an ultrasound image based on the synthesized signal subjected to the amplitude detection and the log compression (step S8).

After the ultrasound image is generated, the display control function 532 displays the generated ultrasound image (step S9).

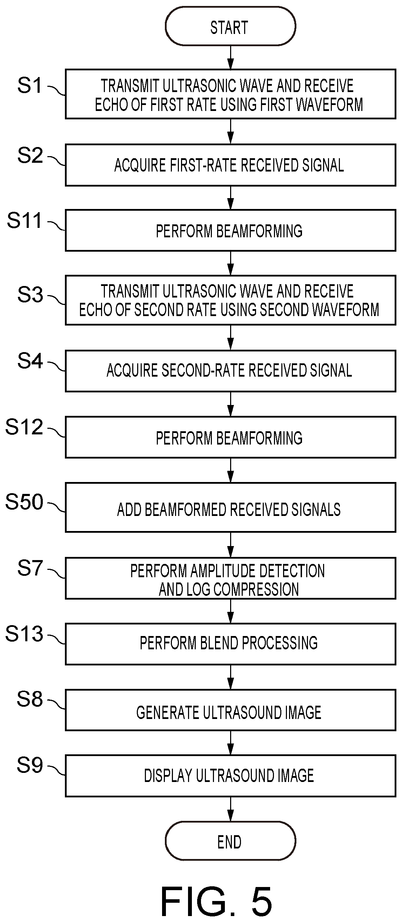

Next, an operation example of an ultrasound diagnostic apparatus according to a first comparative example will be described, with a focus on differences from FIG. 2. FIG. 5 is a flow chart showing an operation example of the ultrasound diagnostic apparatus according to the first comparative example.

As shown in FIG. 5, in the first comparative example, after the first-rate received signal is acquired (step S2), beamforming is performed on the acquired first-rate received signal (step S11).

In addition, in the first comparative example, after the second-rate received signal is acquired (step S4), beamforming is performed on the acquired second-rate received signal (step S12).

After beamforming is performed on the second-rate received signal, the beamformed first-rate received signal and the beamformed second-rate received signal are added to generate a synthesized signal (step S50).

In addition, in the first comparative example, after amplitude detection and log compression are performed (step S7), blend processing involving adjusting proportions of the harmonic component and the fundamental wave component according to depth is performed (step S13). The generation of an ultrasound image (step S8) is performed using the synthesized signal after the blend processing.

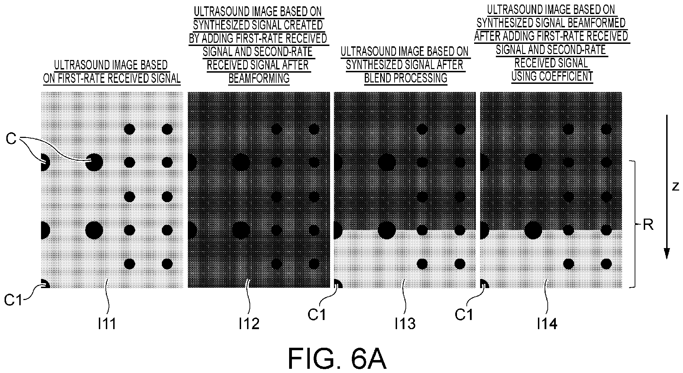

FIG. 6A is a diagram showing an ultrasound image in the operation example of the ultrasound diagnostic apparatus 1 according to the first embodiment. FIG. 6A shows four ultrasound images I11, I12, 113, and I14 as ultrasound images (in other words, B-mode images) of a cyst phantom simulating a cyst C.

A first ultrasound image I11 is an ultrasound image generated using only the first-rate received signal. A second ultrasound image I12 is an ultrasound image generated using a synthesized signal created by adding the first-rate received signal and the second-rate received signal after beamforming (BF). In the second ultrasound image I12, since brightness is insufficient, for example, a deep cyst C1 is not appropriately displayed. A third ultrasound image I13 is an ultrasound image generated using a synthesized signal created by adding the first-rate received signal and the second-rate received signal after beamforming and then performing blend processing. The third ultrasound image I13 is an ultrasound image generated according to the first comparative example. A fourth ultrasound image I14 is an ultrasound image generated based on a synthesized signal having been beamformed after adding the first-rate received signal and the second-rate received signal using a coefficient. In other words, the fourth ultrasound image I14 is an ultrasound image generated using a signal created by beamforming a synthesized signal. The fourth ultrasound image I14 is an ultrasound image generated according to the first embodiment. The fourth ultrasound image I14 can include a region R in which the harmonic component and the fundamental wave component are blended in proportions according to the depth z in a similar manner to the third ultrasound image I13. Although the illustration in FIG. 6A is simplified, an image within the region R in the fourth ultrasound image I14 may be an image of which brightness gradually increases as the depth z increases.

As shown in FIG. 5, to obtain the harmonic component by adding the beamformed first-rate received signal and the beamformed second-rate received signal, beamforming must be performed for each received signal. Therefore, the computational load of beamforming increases. In contrast, according to the first embodiment, by applying beamforming to the synthesized signal generated by adding the first-rate received signal and the second-rate received signal, the same image quality as in the first comparative example can be achieved while halving the number of beamforming operations. Therefore, the computational load of beamforming can be reduced by approximately 50%. In addition, according to the first embodiment, since there is no longer a need to perform blend processing of adjusting the proportions of the harmonic component and the fundamental wave component according to depth, the processing load required for the overall display of ultrasound images can also be reduced.

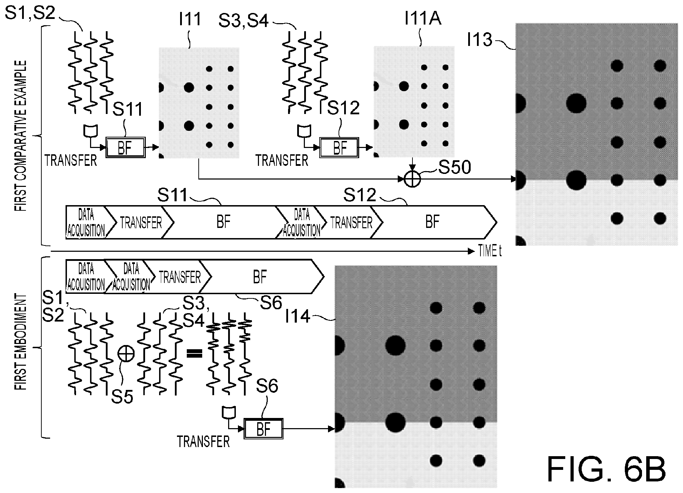

FIG. 6B is an explanatory diagram for describing a frame rate in the operation example of the ultrasound diagnostic apparatus 1 according to the first embodiment. More specifically, FIG. 6B is an explanatory diagram for comparing and describing, from the perspective of frame rate, the operation of the ultrasound diagnostic apparatus 1 according to the first embodiment shown in FIG. 2 and the operation of the ultrasound diagnostic apparatus according to the first comparative example shown in FIG. 5. As shown in FIG. 6B, in the first comparative example, after data of the first-rate received signal is acquired and transferred to a beamformer of the reception circuitry, a first beamforming (step S11) is performed by a beamformer that is hardware. Beamforming using hardware is also called hardware beamforming (HWBF). Subsequently, in the first comparative example, after data of the second-rate received signal is acquired and transferred to a beamformer, a second beamforming (step S12) is performed in the beamformer. Then, first-rate data representing the first beamforming and second-rate data representing the second beamforming are synthesized (step S50).

In contrast, as shown in FIG. 6B, in the first embodiment, data of the first-rate received signal and data of the second-rate received signal are synthesized (step S5), and beamforming by the beamforming function 530 that is a function of hardware 53 realized using software is performed with respect to the synthesized data (step S6). Beamforming using software is also called software beamforming (SWBF).

Computational requirements for beamforming affect frame rate. When beamforming is performed twice as in the first comparative example, since the computational requirements for beamforming increase, the frame rate decreases. According to the first embodiment, since beamforming need only be performed once, a decrease in the frame rate due to an increase in computational requirements can be suppressed. In other words, according to the first embodiment, the frame rate can be improved. Note that the beamforming performed by the ultrasound diagnostic apparatus 1 according to the present embodiment is not limited to software beamforming and may be hardware beamforming.

As described above, in the first embodiment, the signal acquirer 511 acquires, by transmitting/receiving ultrasonic waves with different waveforms a plurality of times in a same direction, a plurality of ultrasonic signals corresponding to the plurality of transmissions/receptions, respectively. In addition, the synthesizer 512 generates a synthesized signal by synthesizing the plurality of ultrasonic signals acquired by the signal acquirer 511 using a coefficient that varies in a depth direction before beamforming. Furthermore, the beamforming function 530 performs beamforming on the synthesized signal generated by the synthesizer 512.

Accordingly, since beamforming need no longer be performed for each ultrasonic signal, the processing load of beamforming can be reduced and the frame rate can be improved. Since the processing load can be reduced, general-purpose operational circuitry such as CPUs and GPUs can be used. In addition, since a data volume of channel signals that must be sent to the beamforming function 530 can be reduced, the time or a memory volume required for data transfer to the beamforming function 530 can be reduced. Furthermore, by subjecting a synthesized signal of which the amplitude has been reduced as compared to the amplitude of received signals (in other words, fundamental wave signals) to beamforming, an acceptable dynamic range of a processing block after beamforming can be reduced. Therefore, processing efficiency of displaying ultrasound images can be improved while reducing costs.

In addition, in the first embodiment, the image generation function 531 generates an ultrasound image based on the synthesized signal subjected to beamforming by the beamforming function 530.

Accordingly, since an ultrasound image can be generated based on the synthesized signal after beamforming, the processing load of generating an ultrasound image can be reduced.

Furthermore, in the first embodiment, in the plurality of transmissions/receptions, the signal acquirer 511 transmits/receives ultrasonic waves modulated with respect to previously transmitted/received ultrasonic waves.

Accordingly, a harmonic component for harmonic imaging can be appropriately generated.

In addition, in the first embodiment, in the plurality of transmissions/receptions, the signal acquirer 511 transmits/receives ultrasonic waves modulated with respect to previously transmitted/received ultrasonic waves by transmitting/receiving ultrasonic waves of which phases have been changed with respect to previously transmitted/received ultrasonic waves.

Accordingly, a harmonic component can be easily and appropriately generated.

Furthermore, in the first embodiment, in the plurality of transmissions/receptions, the signal acquirer 511 may transmit/receive ultrasonic waves modulated with respect to previously transmitted/received ultrasonic waves by transmitting/receiving ultrasonic waves of which amplitudes have been changed with respect to previously transmitted/received ultrasonic waves.

Accordingly, a harmonic component can be easily and appropriately generated.

In addition, in the first embodiment, the synthesizer 512 synthesizes a plurality of ultrasonic signals by adding the plurality of ultrasonic signals.

Accordingly, a synthesized signal can be easily generated.

Furthermore, in the first embodiment, the synthesizer 512 may synthesize a plurality of ultrasonic signals by subtracting the plurality of ultrasonic signals.

Accordingly, a synthesized signal can be easily generated.

In addition, in the first embodiment, the synthesizer 512 synthesizes a plurality of ultrasonic signals using a coefficient so that proportions of a harmonic component and a fundamental wave component included in the synthesized signal vary depending on depth.

Accordingly, an ultrasound image with favorable image quality in accordance with depth can be generated.

Furthermore, in the first embodiment, the synthesizer 512 synthesizes a plurality of ultrasonic signals so that a proportion of a fundamental wave component becomes higher than a proportion of a harmonic component in a synthesized signal of a deep portion but the proportion of the harmonic component becomes higher than the proportion of the fundamental wave component in a synthesized signal of a shallow portion.

Accordingly, an ultrasound image with high image quality that balances sensitivity in a deep portion with contrast and resolution in a shallow portion can be generated.

In addition, in the first embodiment, the synthesizer 512 generates synthesized signals according to the mathematical expression presented above.

Accordingly, a synthesized signal in which the proportion of the fundamental wave component increases as the depth increases and the proportion of the harmonic component increases as the depth decreases can be appropriately generated.

### (Modification)

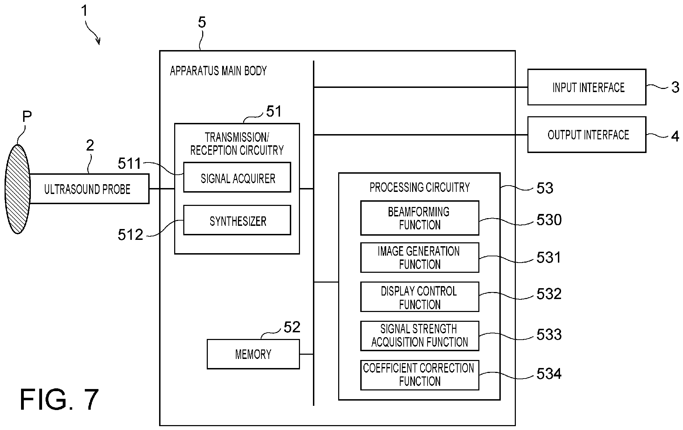

Next, a modification of the first embodiment in which a coefficient is varied according to signal strength will be described with a focus on differences from the embodiment described above. FIG. 7 is a block diagram showing a configuration of the ultrasound diagnostic apparatus 1 according to the modification of the first embodiment.

In the example shown in FIG. 7, the processing circuitry 53 further includes a signal strength acquisition function 533 and a coefficient correction function 534 in addition to the components shown in FIG. 1B. The signal strength acquisition function 533 and the coefficient correction function 534 may be included in the transmission/reception circuitry 51.

The signal strength acquisition function 533 acquires a signal strength of an ultrasonic signal before being synthesized by the synthesizer 512. For example, the signal strength acquisition function 533 calculates a signal strength based on the ultrasonic signal before being synthesized by the synthesizer 512 corresponding to at least one transmission/reception. The signal strength acquisition function 533 may calculate brightness of a received signal as the signal strength.

The coefficient correction function 534 corrects a coefficient used to synthesize ultrasonic signals by the synthesizer 512 based on the signal strength acquired by the signal strength acquisition function 533. The coefficient correction function 534 corrects the coefficient by increasing the coefficient so that, when the signal strength is high, the depth increases when the proportion of the fundamental wave component included in the synthesized signal becomes higher than the proportion of the harmonic component included in the synthesized signal. In addition, the coefficient correction function 534 corrects the coefficient by reducing the coefficient so that, when the signal strength is low, the depth decreases when the proportion of the fundamental wave component included in the synthesized signal becomes higher than the proportion of the harmonic component included in the synthesized signal.

The synthesizer 512 changes a coefficient according to the signal strength acquired by the signal strength acquisition function 533. In the example shown in FIG. 7, the synthesizer 512 changes the coefficient according to the signal strength by synthesizing a plurality of ultrasonic signals before beamforming using the coefficient corrected by the coefficient correction function 534. Specifically, the synthesizer 512 changes the coefficient so that, when the signal strength is high, the depth increases when the proportion of the fundamental wave component included in the synthesized signal becomes higher than the proportion of the harmonic component included in the synthesized signal. In addition, the synthesizer 512 changes the coefficient so that, when the signal strength is low, the depth decreases when the proportion of the fundamental wave component included in the synthesized signal becomes higher than the proportion of the harmonic component included in the synthesized signal.

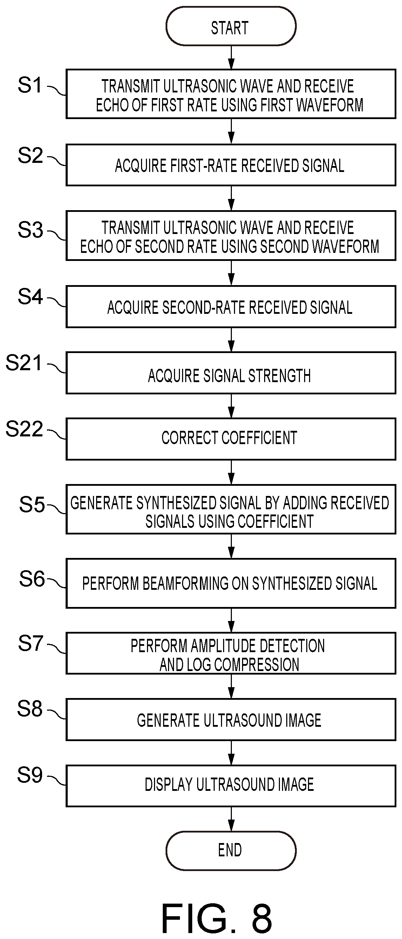

Next, an operation example of the ultrasound diagnostic apparatus 1 according to the modification of the first embodiment configured as described above will be described, with a focus on differences from FIG. 2. FIG. 8 is a flow chart showing an operation example of the ultrasound diagnostic apparatus 1 according to the modification of the first embodiment.

In the example shown in FIG. 8, after the second-rate received signal is acquired (step S4), the signal strength acquisition function 533 acquires a signal strength (step S21). For example, the signal strength acquisition function 533 calculates the brightness of the first-rate received signal per channel. Alternatively, the signal strength acquisition function 533 may calculate the brightness of the second-rate received signal per channel. Alternatively, the signal strength acquisition function 533 may calculate both the brightness of the first-rate received signal and the brightness of the second-rate received signal per channel. When calculating the brightness of the first-rate received signal, the signal strength acquisition step may be performed immediately after acquiring the first-rate received signal.

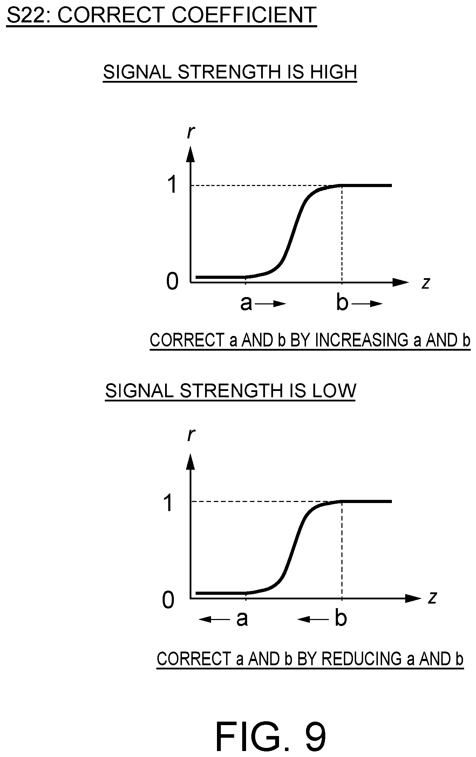

After the signal strength is acquired, the coefficient correction function 534 corrects the coefficient according to the signal strength (step S22). The coefficient may be corrected per channel. FIG. 9 is a diagram showing a coefficient correction step in the operation example of the ultrasound diagnostic apparatus 1 according to the modification of the first embodiment.

In the example shown in FIG. 9, when the signal strength is high (for example, when the signal strength is equal to or higher than a set threshold strength), the coefficient correction function 534 corrects the depths a and b when a mode of transition of the coefficient r according to depth changes by increasing the depths a and b. In other words, the coefficient correction function 534 corrects the coefficient by increasing the coefficient so that, when the signal strength is high, the depth increases when the proportion of the fundamental wave component included in the synthesized signal becomes higher than the proportion of the harmonic component included in the synthesized signal. A correction amount of the coefficient by increasing the coefficient may gradually increase or increase in a stepwise manner according to the signal strength. When the signal strength is high, since a synthesized signal in a relatively deep portion also has sufficient strength, the coefficient correction function 534 corrects the coefficient by increasing the coefficient so that only the fundamental wave component with high strength is present at deeper locations.

In addition, in the example shown in FIG. 9, when the signal strength is low (for example, when the signal strength is equal to lower than a set threshold strength), the coefficient correction function 534 corrects the depths a and b when a mode of transition of the coefficient r according to depth changes by reducing the depths a and b. In other words, the coefficient correction function 534 corrects the coefficient by reducing the coefficient so that, when the signal strength is low, the depth decreases when the proportion of the fundamental wave component included in the synthesized signal becomes higher than the proportion of the harmonic component included in the synthesized signal. A correction amount of the coefficient by reducing the coefficient may gradually increase or increase in a stepwise manner according to the signal strength. When the signal strength is low, since the strength of a synthesized signal in a relatively shallow portion is also insufficient, the coefficient correction function 534 corrects the coefficient by reducing the coefficient so that the fundamental wave component with high strength is present at shallower locations.

After the coefficient is corrected, as shown in FIG. 8, the synthesizer 512 generates a synthesized signal by adding the first-rate received signal and the second-rate received signal using the corrected coefficient (step S5).

As described above, in the modification of the first embodiment, the signal strength acquisition function 533 acquires a signal strength of an ultrasonic signal before being synthesized by the synthesizer 512. The synthesizer 512 changes a coefficient according to the signal strength acquired by the signal strength acquisition function 533.

Accordingly, since an ultrasonic signal before beamforming can be synthesized using an appropriate coefficient according to the signal strength, the image quality of the ultrasound image can be further improved.

In addition, in the modification of the first embodiment, the synthesizer 512 changes the coefficient so that, when the signal strength is high, the depth increases when the proportion of the fundamental wave component included in the synthesized signal becomes higher than the proportion of the harmonic component included in the synthesized signal, and when the signal strength is low, the depth decreases when the proportion of the fundamental wave component becomes higher than the proportion of the harmonic component.

Accordingly, since an ultrasonic signal before beamforming can be synthesized more appropriately using a coefficient according to the signal strength, the image quality of the ultrasound image can be further improved.

### (Second embodiment)

Next, a second embodiment of generating a synthesized signal further using gain will be described with a focus on differences from the embodiment described above.

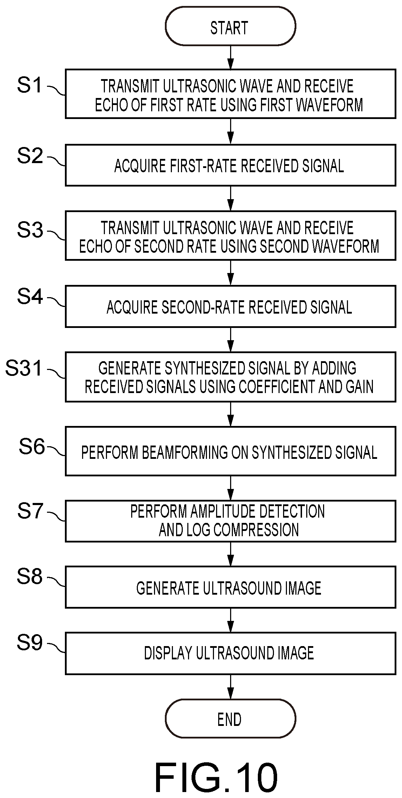

FIG. 10 is a flow chart showing an operation example of the ultrasound diagnostic apparatus 1 according to the second embodiment. An example of synthesizing a plurality of ultrasonic signals before beamforming using a coefficient has been described above. In contrast, in the second embodiment, a plurality of ultrasonic signals before beamforming are synthesized further using a gain. Specifically, the synthesizer 512 synthesizes a plurality of ultrasonic signals by applying a gain to at least one ultrasonic signal among the plurality of ultrasonic signals. More specifically, in the example shown in FIG. 10, after the second-rate received signal is acquired (step S4), the synthesizer 512 generates a synthesized signal by adding the first-rate received signal and the second-rate received signal using a coefficient and the gain (step S31).

In the example shown in FIG. 10, the synthesizer 512 adds the first-rate received signal and the second-rate received signal according to the mathematical expression below. [Expression 2] $\begin{matrix}{s}_{sum} \left(z, θ, m\right) = \left\{r\left(z\right)+G∗\left(1-r\left(z\right)\right)\right\} ∗ {s}_{1} \left(z, θ, m\right) \\ + G ∗ \left(1-r\left(z\right)\right) ∗ {s}_{2} \left(z, θ, m\right) \\ r \left(z\right) = \left\{\begin{matrix}0 & \left(z<a\right) \\ 1 - \frac{1 + cos \left(\frac{z - a}{b - a}∗π\right)}{2} & \left(a\leq z\leq b\right) \\ 1 & \left(z>b\right)\end{matrix}\right.\end{matrix}$

In the above mathematical expression, reference sign G denotes gain (the same shall apply hereinafter).

According to the above mathematical expression, the coefficient r is 0 in an interval where the depth is less than a. In this case, since sₛᵤₘ = Gs₁ + Gs₂, the synthesized signal corresponds to the harmonic signal (s₁ + s₂) multiplied by the gain G. Consequently, in shallow intervals, only a harmonic signal with increased brightness is present.

In addition, according to the above mathematical expression, the coefficient r is 1-(1 + cos((z-a)/(b-a)*π))/2 in an interval where the depth is equal to or more than a and equal to or less than b. In other words, in an interval where the depth is equal to or more than a and equal to or less than b, the coefficient r takes values equal to or more than 0 and equal to or less than 1 and increases as the depth increases. In an interval where the depth is equal to or more than a and equal to or less than b, since sₛᵤₘ = G(1-r)(s₁ + s₂) + rs₁, the interval contains a mixture of the harmonic components (s₁ + s₂) multiplied by the gain G(1-r) and the fundamental wave component (s₁) multiplied by the coefficient r.

Furthermore, according to the above mathematical expression, the coefficient r is 1 in an interval where the depth exceeds b. In this case, since sₛᵤₘ = s₁, the synthesized signal is the first-rate received signal or, in other words, the fundamental wave signal. Consequently, in deep intervals, only the fundamental wave component is present.

After the synthesized signal is generated, as shown in FIG. 10, the beamforming function 530 performs beamforming on the synthesized signal (step S6).

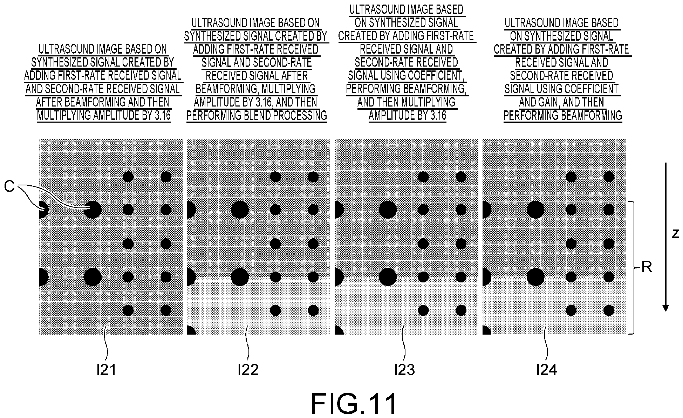

FIG. 11 is a diagram showing an ultrasound image in the operation example of the ultrasound diagnostic apparatus 1 according to the second embodiment. FIG. 11 shows four ultrasound images I21, I22, I23, and I24 as ultrasound images of a cyst phantom. A first ultrasound image I21 is an ultrasound image generated using a synthesized signal created by adding the first-rate received signal and the second-rate received signal after beamforming and then multiplying an amplitude by 3.16. In other words, the first ultrasound image I21 is an ultrasound image generated using a synthesized signal created by adding the first-rate received signal and the second-rate received signal after beamforming and then applying a gain of 10 dB. A second ultrasound image I22 is an ultrasound image generated using a synthesized signal created by adding the first-rate received signal and the second-rate received signal after beamforming, multiplying an amplitude by 3.16, and then performing blend processing. A third ultrasound image I23 is an ultrasound image generated using a synthesized signal having been beamformed after adding the first-rate received signal and the second-rate received signal using a coefficient and then multiplying an amplitude by 3.16. A fourth ultrasound image I24 is an ultrasound image generated using a synthesized signal having been beamformed after adding the first-rate received signal and the second-rate received signal using a coefficient and a gain. The fourth ultrasound image I24 is an ultrasound image generated according to the second embodiment. In the fourth ultrasound image I24, a variation in brightness across the entire image is reduced compared to the fourth ultrasound image I14 described in the first embodiment.

An amplitude of a second harmonic signal is approximately 10 dB lower than the amplitude of a fundamental wave signal. Therefore, the brightness of the synthesized signal may increase in a portion where the proportion of the fundamental wave component is high, which may result in reduced brightness uniformity across the entire ultrasound image.

In contrast, according to the second embodiment, the synthesizer 512 can improve the brightness of shallow portions by synthesizing a plurality of ultrasonic signals further using a gain. Accordingly, brightness uniformity across the entire ultrasound image can be improved.

### (Third embodiment)

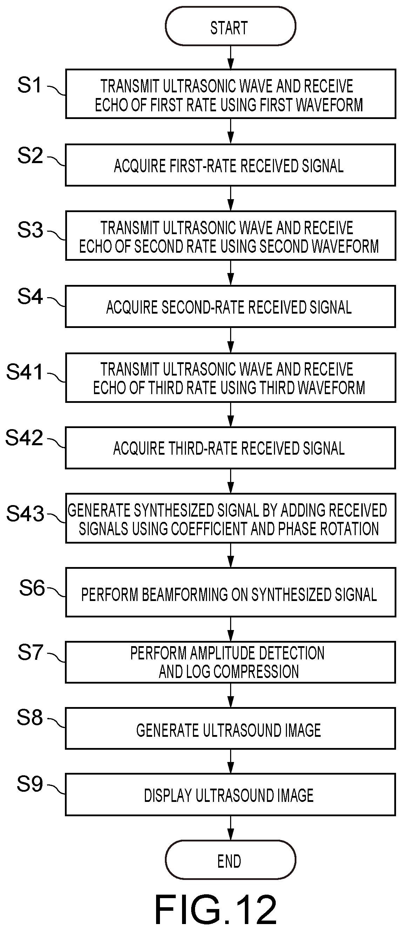

Next, a third embodiment of generating a synthesized image further using phase rotation will be described with a focus on differences from the embodiments described above. FIG. 12 is a flow chart showing an operation example of the ultrasound diagnostic apparatus 1 according to the third embodiment.

In the third embodiment, the synthesizer 512 synthesizes a plurality of ultrasonic signals before beamforming further using phase rotation of ultrasonic signals in addition to a coefficient. Specifically, in the example shown in FIG. 12, after acquiring the second-rate received signal, the signal acquirer 511 transmits an ultrasonic wave and receives an echo of a third rate using a third waveform via the ultrasound probe 2 (step S41). Note that the transmission/reception of the second rate is performed by shifting a phase of a transmission waveform by 120 degrees relative to the transmission/reception of the first rate. In addition, the transmission/reception of the third rate is performed by shifting the phase of the transmission waveform by 120 degrees relative to the transmission/reception of the second rate.

After transmitting the ultrasonic wave and receiving the echo of the third rate, the signal acquirer 511 acquires a third-rate received signal based on the received echo (step S42).

After acquiring the third-rate received signal, the synthesizer 512 generates a synthesized signal by adding the first-rate received signal, the second-rate received signal, and the third-rate received signal using a coefficient that varies in the depth direction and phase rotation (step S43).

In the example shown in FIG. 12, the synthesizer 512 adds the first-rate received signal, the second-rate received signal, and the third-rate received signal according to the mathematical expression below. [Expression 3] $\begin{matrix}{s}_{sum} \left(z, θ, m\right) = {s}_{1} \left(z, θ, m\right) + exp \left\{j\left(π⋅\frac{2}{3}⋅r\left(z\right)\right)\right\} ⋅ {s}_{2} \left(z, θ, m\right) \\ + exp \left\{-j\left(π⋅\frac{2}{3}⋅r\left(z\right)\right)\right\} ⋅ {s}_{3} \left(z, θ, m\right) \\ r \left(z\right) = \left\{\begin{matrix}0 & \left(z<a\right) \\ 1 - \frac{1 + cos \left(\frac{z - a}{b - a}∗π\right)}{2} & \left(a\leq z\leq b\right) \\ 1 & \left(z>b\right)\end{matrix}\right.\end{matrix}$

According to the above mathematical expression, the coefficient r is 0 in an interval where the depth is less than a. In this case, according to sₛᵤₘ=s₁ + s₂ + s₃, the synthesized signal is a simple sum of three received signals that differ in phase by 120 degrees. In other words, the synthesized signal is a third-order harmonic signal (s₁ + s₂ + _{S3}). Consequently, in shallow intervals, only the third-order harmonic component is present.

In addition, according to the above mathematical expression, the coefficient r is 1-(1 + cos((z-a)/(b-a)*n))/2 in an interval where the depth is equal to or more than a and equal to or less than b. In other words, in an interval where the depth is equal to or more than a and equal to or less than b, the coefficient r takes values equal to or more than 0 and equal to or less than 1 and increases as the depth increases. In an interval where the depth is equal to or more than a and equal to or less than b, a mixture of the third-order harmonic component and the second-order harmonic component is present. Furthermore, according to the above mathematical expression, the coefficient r is 1 in an interval where the depth exceeds b. In this case, sₛᵤₘ = s₁ + exp{j(π*2/3)}s₂ + exp{-j(π*2/3)}s₃ is true. In other words, in an interval where the depth exceeds a, with respect to a combination where phases of the three received signals s1, s2, and s3 before phase rotation are θ°, θ+120°, and θ+240°, respectively, phase rotation is performed using a combination where values of rotation phase angles are 0°, 120°, and 240°. A second-order harmonic signal can be generated by performing such a phase rotation.

After the synthesized signal is generated, as shown in FIG. 12, the beamforming function 530 performs beamforming on the synthesized signal (step S6).

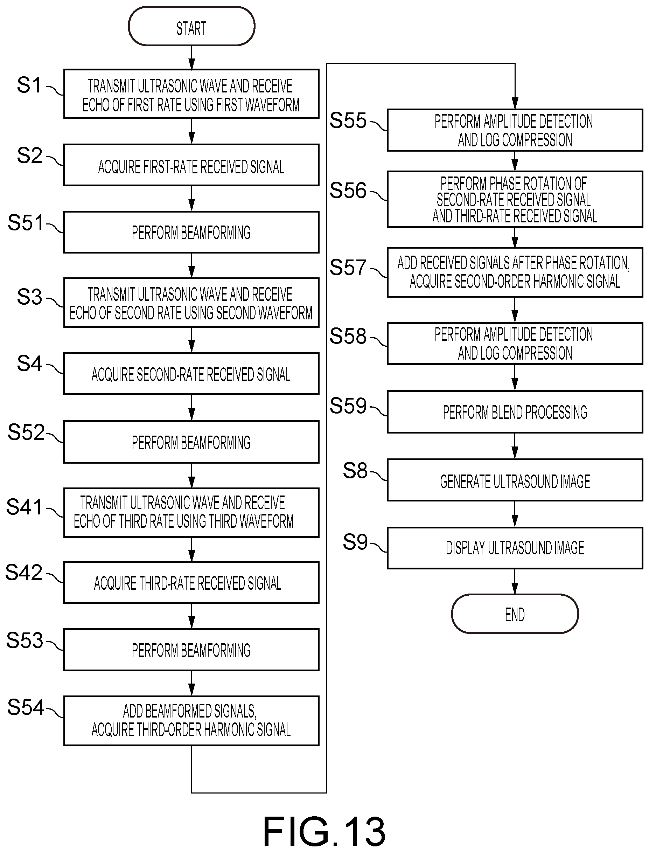

Next, an operation example of an ultrasound diagnostic apparatus according to a second comparative example will be described, with a focus on differences from FIG. 12. FIG. 13 is a flow chart showing an operation example of the ultrasound diagnostic apparatus according to the second comparative example.

As shown in FIG. 13, in the second comparative example, after the first-rate received signal is acquired (step S2), beamforming is performed on the acquired first-rate received signal (step S51).

In addition, in the second comparative example, after the second-rate received signal is acquired (step S4), beamforming is performed on the acquired second-rate received signal (step S52).

After performing beamforming on the second-rate received signal, an ultrasonic wave of a third rate is transmitted and an echo of the third rate is received using a third waveform via the ultrasound probe 2 (step S41).

After transmitting the ultrasonic wave and receiving the echo of the third rate, a third-rate received signal is acquired based on the received echo (step S42).

After the third-rate received signal is acquired, beamforming is performed on the acquired third-rate received signal (step S53).

After beamforming is performed on the third-rate received signal, the beamformed first-rate received signal, the beamformed second-rate received signal, and the beamformed third-rate received signal are added (step S54). Accordingly, a third-order harmonic signal is acquired.

After the third-order harmonic signal is acquired, amplitude detection and log compression are performed with respect to the acquired third-order harmonic signal (step S55).

After performing the amplitude detection and log compression with respect to the third-order harmonic signal, 120°-phase rotation of the second-rate received signal and -120°-phase rotation of the third-rate received signal are performed (step S56).

After performing the phase rotations, the second-rate received signal and the third-rate received signal are added (step S57). Accordingly, a second-order harmonic signal is acquired.

After the second-order harmonic signal is acquired, amplitude detection and log compression are performed with respect to the acquired second-order harmonic signal (step S58).

After performing the amplitude detection and log compression with respect to the second-order harmonic signal, blend processing of the third-order harmonic signal and the second-order harmonic signal is performed (step S59). The generation of an ultrasound image (step S8) is performed using the signal after the blend processing.

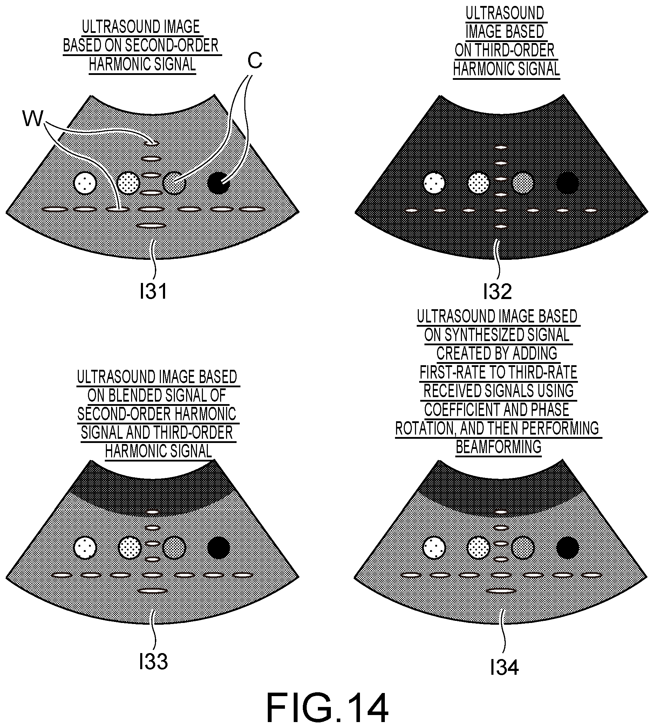

FIG. 14 is a diagram showing an ultrasound image in the operation example of the ultrasound diagnostic apparatus 1 according to the third embodiment. FIG. 14 shows four ultrasound images I31, I32, I33, and I34 as ultrasound images of a cyst phantom including a wire W. A first ultrasound image I31 is an ultrasound image generated using only the second-order harmonic signal. A second ultrasound image I32 is an ultrasound image generated using only the third-order harmonic signal. A third ultrasound image I33 is an ultrasound image generated using a blended signal of the second-order harmonic signal and the third-order harmonic signal. The third ultrasound image I33 is an ultrasound image generated according to the second comparative example. In the first ultrasound image I31, a width of the wire W is large due to poor azimuth resolution. In the second ultrasound image I32, while the azimuth resolution is favorable, sensitivity in deep portions is poor. In contrast, in the third ultrasound image I33, both azimuth resolution and sensitivity in deep portions are favorable. A fourth ultrasound image I34 is an ultrasound image generated using a synthesized signal having been beamformed after adding the first-rate to third-rate received signals using a coefficient and phase rotation. The fourth ultrasound image I34 is an ultrasound image generated according to the third embodiment. The fourth ultrasound image I34 can include a second-order harmonic component or a third-order harmonic component according to the depth z in a similar manner to the third ultrasound image I33. Therefore, the fourth ultrasound image I34 has favorable image quality in a similar manner to the third ultrasound image I33.

As shown in FIG. 13, to obtain the harmonic component by adding the beamformed first-rate to third-rate received signals, beamforming must be performed for each received signal. Therefore, the computational load of beamforming increases. In contrast, according to the third embodiment, by applying beamforming to the synthesized signal generated by adding the first-rate to third-rate received signals, the same image quality as in the second comparative example can be achieved while reducing the number of beamforming operations to one-third. Therefore, the computational load of beamforming can be reduced by approximately 66%. In addition, according to the third embodiment, since there is no longer a need to perform blend processing or phase rotation independent of addition, the processing load required for the overall display of ultrasound images can also be reduced.

Thus far, examples in which received signals of up to the third rate are used as a plurality of ultrasonic signals to generate a synthesized signal, and beamforming is then performed on the generated synthesized signal have been described. The present embodiment is not limited to such examples and may be configured to perform beamforming on a synthesized signal created by synthesizing received signals of up to a fourth or higher rate.

The term "processor" as used in the description given above means, for example, a central processing unit (CPU), a graphics processing unit (GPU), or circuitry such as an application specific integrated circuit (ASIC) or a programmable logic device (for example, a simple programmable logic device (SPLD), a complex programmable logic device (CPLD), or a field programmable gate array (FPGA)). The processor realizes functions by reading and executing programs stored in storage circuitry. Note that a configuration may be adopted in which a program is directly incorporated into the circuitry of the processor instead of being stored in the storage circuitry. In this case, the processor realizes functions by reading and executing the program incorporated into the circuitry. The processor is not limited to a single circuitry configuration of the processor and a plurality of independent circuitries may be combined to constitute a single processor and the functions may be realized by the single processor. Furthermore, the plurality of constituent elements in FIG. 1A may be integrated into a single processor and the functions may be realized by the single processor.

According to at least one of the embodiments described above, processing load of beamforming can be reduced.

While certain embodiments have been described above, the embodiments have been presented by way of example only and are not intended to limit the scope of the invention. The novel device and method described in the present specification can be implemented in various other modes. In addition, various omissions, substitutions, and modifications can be made to the modes of the device and method described in the present specification to the extent that they do not depart from the scope of the invention as defined by the appended claims. The appended claims are intended to include such modes and modifications that are included in the scope of the invention.

## Claims

1. An ultrasonic signal processing apparatus, comprising:
an acquirer configured to acquire, by transmitting/receiving ultrasonic waves with different waveforms a plurality of times in a same direction, a plurality of ultrasonic signals corresponding to the plurality of transmissions/receptions, respectively;
a synthesizer configured to generate a synthesized signal by synthesizing the plurality of ultrasonic signals acquired by the acquirer using a coefficient that varies in a depth direction before beamforming; and
a processing circuitry configured to perform the beamforming on the synthesized signal generated by the synthesizer.

2. The ultrasonic signal processing apparatus according to claim 1, wherein the processing circuitry is further configured to generate an ultrasound image based on the synthesized signal subjected to the beamforming.

3. The ultrasonic signal processing apparatus according to claim 1, wherein the acquirer is further configured to transmit/receive, in the plurality of transmissions/receptions, ultrasonic waves modulated with respect to previously transmitted/received ultrasonic waves by transmitting/receiving ultrasonic waves of which phases or amplitudes have been changed with respect to the previously transmitted/received ultrasonic waves.

4. The ultrasonic signal processing apparatus according to claim 1, wherein the synthesizer is further configured to synthesize the plurality of ultrasonic signals by adding or subtracting the plurality of ultrasonic signals.

5. The ultrasonic signal processing apparatus according to claim 1, wherein the synthesizer is further configured to synthesize the plurality of ultrasonic signals using the coefficient so that proportions of a harmonic component and a fundamental wave component included in the synthesized signal vary depending on depth.

6. The ultrasonic signal processing apparatus according to claim 5, wherein the synthesizer is further configured to synthesize the plurality of ultrasonic signals so that a proportion of the fundamental wave component becomes higher than a proportion of the harmonic component in the synthesized signal of a deep portion but the proportion of the harmonic component becomes higher than the proportion of the fundamental wave component in the synthesized signal of a shallow portion.

7. The ultrasonic signal processing apparatus according to claim 5, wherein the synthesizer is further configured to synthesize the plurality of ultrasonic signals by further using a gain.

8. The ultrasonic signal processing apparatus according to claim 1, wherein the synthesizer is further configured to synthesize the plurality of ultrasonic signals by further using a phase rotation of the ultrasonic signal.

9. The ultrasonic signal processing apparatus according to claim 8, wherein the synthesizer is further configured to synthesize the plurality of ultrasonic signals using the coefficient and the phase rotation so that proportions of a second-order harmonic component and a third-order harmonic component included in the synthesized signal vary depending on depth.

10. The ultrasonic signal processing apparatus according to claim 9, wherein the synthesizer is further configured to synthesize the plurality of ultrasonic signals so that a proportion of the second-order harmonic component becomes higher than a proportion of the third-order harmonic component in the synthesized signal of a deep portion but the proportion of the third-order harmonic component becomes higher than the proportion of the second-order harmonic component in the synthesized signal of a shallow portion.

11. The ultrasonic signal processing apparatus according to any one of claims 1 to 10, wherein
the processing circuitry is further configured to acquire a signal strength of the ultrasonic signal before being synthesized by the synthesizer, and
the synthesizer is further configured to change the coefficient according to the signal strength acquired by the processing circuitry.

12. The ultrasonic signal processing apparatus according to claim 11, wherein the synthesizer is further configured to change the coefficient so that, when the signal strength is high, depth increases when the proportion of the fundamental wave component included in the synthesized signal becomes higher than the proportion of the harmonic component included in the synthesized signal, and when the signal strength is low, the depth decreases when the proportion of the fundamental wave component becomes higher than the proportion of the harmonic component.

13. An ultrasound diagnostic apparatus, comprising:
the ultrasonic signal processing apparatus according to any one of claims 1 to 12; and
an ultrasound probe used in a plurality of transmissions/receptions of the ultrasonic waves.

14. An ultrasonic signal processing method, comprising:
acquiring, by transmitting/receiving ultrasonic waves with different waveforms a plurality of times in a same direction, a plurality of ultrasonic signals corresponding to the plurality of transmissions/receptions, respectively;
generating a synthesized signal by synthesizing the plurality of acquired ultrasonic signals using a coefficient that varies in a depth direction before beamforming; and
performing the beamforming on the generated synthesized signal.

15. A program for causing a computer to execute:
a procedure for acquiring, by transmitting/receiving ultrasonic waves with different waveforms a plurality of times in a same direction, a plurality of ultrasonic signals corresponding to the plurality of transmissions/receptions, respectively;
a procedure for generating a synthesized signal by synthesizing the plurality of acquired ultrasonic signals using a coefficient that varies in a depth direction before beamforming; and
a procedure for performing the beamforming on the generated synthesized signal.
